# EUROPÄISCHE PATENTSCHRIFT

(11) **EP 1 357 926 B1**
(45) Veröffentlichungstag und Bekanntmachung des Hinweises auf die Patenterteilung: **14.07.2004**
(21) Anmeldenummer: 02708270.0
(22) Anmeldetag: 10.01.2002
(51) Int. Cl.: A61K 35/78, A61P 17/02

(54) **TOPISCHE VERWENDUNG VON ÄTHERISCHEN ÖLEN ZUR BEHANDLUNG VON WUNDHEILUNGSSTÖRUNGEN**
LOCAL USE OF ETHEREAL OILS
UTILISATION TOPIQUE D'HUILES ESSENTIELLES

(30) Priorität: 10.01.2001 DE 10100811
(43) Veröffentlichungstag der Anmeldung: 05.11.2003
(73) Patentinhaber: Walther Schoenenberger Pflanzensaftwerk GmbH & Co. KG, 71106 Magstadt (DE); Heiss, Markus M., 82343 Pöcking (DE); Tarabichi, Anwar, 82205 Gilching (DE)
(72) Erfinder: HEISS, Markus, M., 82343 Pöcking (DE); TARABICHI, Anwar, 82205 Gilching (DE); GREITHER, Otto, 83052 Bruckmühl (DE)
(74) Vertreter: VOSSIUS & PARTNER
(86) Internationale Anmeldenummer: PCT/EP2002/000185
(87) Internationale Veröffentlichungsnummer: WO 2002/055096

(56) Entgegenhaltungen:
- EP-A- 0 737 479
- FR-A- 2 662 939
- US-A1- 2002 037 312

## Beschreibung

Diese Erfindung betrifft die topische Verwendung von ätherischen Ölen und ihrer hochprozentigen lipophilen und hydrophilen Zubereitungen zur topischen Therapie oder Prophylaxe von Wundheilungsstörungen, unterstützt durch ihre Gewebsgängigkeit und Zerstörung der Keime bei gleichzeitiger Schonung des Gewebes.

Die Behandlung der Wundheilungsstörung ist auch heute noch eine Herausforderung der modernen Medizin. Während die physiologische Wundheilung vier klar definierte Phasen durchläuft, gestaltet sich die sekundäre Wundheilung und die Heilung infizierter Wunden problematisch. Von chronischen Wunden sind in der Bundesrepublik Deutschland alleine bis zu 2 Millionen Menschen betroffen. Die aufwendige Behandlung verschlingt jährlich nahezu fünf Milliarden Mark, zudem ist ein hoher Prozentsatz der chronischen Wunden therapierefraktär bzw. neigt zu Rezidivierung.

**Eine Wunde stellt per se kein Krankheitsbild dar.** Unter dem Begriff Wunde ist dabei eine Läsion bzw. eine umschriebene Störung des Aufbaus und der Struktur des schützenden äußeren Organs (Haut und Schleimhäute mit den dazugehörenden Anhangsgebilden) und den darunterliegenden Weichteilen (Fett- und Muskelschicht) zu verstehen. Diese Läsion beinhaltet eine Unterbrechung der Integrität des Gewebes mit oder ohne Substanz- und Gewebeverlust. Der Großteil der Wunden entsteht durch traumatische Noxen.

Die physiologische Wundheilung, die unmittelbar nach Entstehung der Wunde einsetzt, ist pathobiochemisch und pathophysiologisch in ihrem Ablauf weitgehend aufgeklärt. Ihre vier Phasen werden in den einschlägigen Lehrbüchern wie folgt beschrieben:
1. Exsudative Phase, beginnend unmittelbar nach der Entstehung der Wunde mit einer Dauer von bis zu 3 Tagen,
2. Proliferative Phase, mit Formation des Granulationsgewebes, anschließend an die 1. Phase mit einer Dauer von bis zu 12 Tagen,
3. Reparative Phase, mit Remodelierung und Formation der Matrix, sowie
4. Maturation des Epithels mit Differenzierung bzw, Narbenbildung.

Dagegen liegt eine Wundheilungsstörung (siehe unter anderem (5,16)) dann vor, wenn eine Wunde nicht nach dem physiologischen Ablauf der Wundheilung in angemessener Zeitspanne mit oder ohne Narbenbildung abheilt. Unter einer schlecht heilenden Wunde versteht der Fachmann jede Art von Wunde, die nicht innerhalb der physiologischen Abheilungszeit von 2-3 Wochen wieder abheilt.

Die gestörte Wundheilung mit der Entwicklung einer chronischen Wunde ist als ein Krankheitssyndrom anzusehen. Dieses Syndrom ist mit komplexen gesundheitlichen Störungen und Komplikationsrisiken behaftet. Die Wundheilungsstörung kann monooder multifaktoriell bedingt sein.

Diese krankhafte chronische Störung ist mit Schmerzen unterschiedlicher Ausprägung behaftet. Die Behandlung kann lange Hospitalisierung und Immobilisierung des Patienten, hohen medizinischen, pflegerischen und materiellen Aufwand sowie lange Arbeitsunfähigkeitsdauer des Patienten bedingen. Psychosoziale Störungen kommen häufig hinzu. Nicht selten treten Komplikationen durch Fortschreiten und Übergreifen der in der Wunde befindlichen pathogenen Keime auf andere Organe bzw. Organsysteme auf. Irreversible Organschädigung, Defektheilung und Funktionsdefizite können zurückbleiben

Nach bisherigem Stand der Wissen werden endogene und exogene Einfluß- und Risikofaktoren der Wundheilungsstörung definiert.

Zu den zahlreichen endogenen Einflußfaktoren zählen in erster Linie:
- Störungen der Durchblutung
- Eiweißmangelsyndrome, mangelhafte / gestörte Ernährung
- Immunmangel-/Immundefektsyndrome
- Vitamin- und Spurenelementmangelsyndrome
- Gerinnungsstörungen (Gerinnungsfaktorenmangel)
- maligne Erkrankungen und sonstige chronische konsumierende Erkrankungen
- psychische und vegetative Störungen
- hormonelle Störungen
- Alterung

Zu den exogenen Einflußfaktoren zählen:
- **Traumatisierung:**: physikalische Noxen: Abhängigkeit der Schädigung vom einwirkenden Gegenstand (stumpf/scharf), vom Einwirkmechanismus und von der Einwirkdauer
Thermische Noxen: Kälte→ Erfrierungen / Hitze → Verbrennungen
Chemische Noxen: Verätzungen durch Säuren und Laugen
Strahlen: ionisierende Strahlen (α-, β-, γ-Strahlen), UV-Strahlen, thermische Strahlen
- **Pharmaka:**: Glucocorticoide, Zytostatika, Immunsuppressiva, Katecholamine, Colchicin, Penicillamin u.a.
- **Operationen:**: Schnittlänge, sekundäre Traumatisierung
Größe des Eingriffs
Operationsdauer

Nach Neustrukturierung der Einfluß- und Risikofaktoren der Wundheilungsstörung fanden die Erfinder folgende Trias, die bei ihrer quantitativen und qualitativen Ausprägung aber auch in ihrer Entstehung als primäre oder sekundäre Faktoren das große Spektrum endogener und exogener Risikofaktoren umfaßt und Prophylaxe und Therapie der Wundheilungsstörung neu überdenken läßt.

Bei diesen Trias handelt es sich um:
■ **Störung der Durchblutung** im Wundgebiet
■ **Mikrobieller Befall**
■ **Gewebeschaden**

Bei dieser Trias können die Störungen auf qualitativer und/oder quantitativer Ebene vorliegen. Sie können primär bestehen oder sekundär hinzukommen. Die drei Faktoren begünstigen sich gegenseitig.

### Störung der lokalen Blutzirkulation

Zur Verhinderung bzw. Beseitigung einer Wundinfektion durch das Immunsystem sowie zur Reparation und Regeneration des geschädigten Gewebes ist eine suffiziente Blutzirkulation notwendig, Störungen können hier auf drei Ebenen bestehen:
◆ der arteriellen Zufuhr (bei Mikro- oder Makroangiopathie)
◆ der kapillaren Perfusion (bei Zerstörung der Kapillargefäße durch physikalische, chemische oder toxische Noxen, Anstieg des hydrostatischen extravasalen Druckes wie z.B. beim Ödem)
◆ des venösen Abflusses (bei akuter oder chronischer venöser Insuffizienz)

### Mikrobielle Kontamination durch pathogene oder potentiell pathogene Keime

Während die intakte Haut eine Schutzbarriere gegen fast alle Bakterien, Pilze oder sonstige Mikroorganismen bildet, können Keime nach ihrer Schädigung eindringen und sich im Gewebe vermehren. Die Pathogenität der Keime ist nicht nur artbezogen, sondern sie hängt auch von ihrer Quantität, dem Ausmaß des bestehenden Gewebeschadens und von der Anwesenheit weiterer Keime ab. Bei der Herbeiführung einer Infektion bedienen sich Mikroorganismen unter Einsatz verschiedener Enzyme und Toxine mehrerer Mechanismen. Diese sind unter anderem
◆ direktes Eindringen in die Zellen und deren Zerstörung (cytopathischer Effekt)
◆ Schädigung der Verbandsstruktur des Gewebes (Streptokokken)
◆ Anregung der Fibrin-/Kollagenbildung mit Behinderung der Makrophagenmigration (Staphylokokken)
◆ Störung der Regeneration durch Angiogenese und Granulationsgewebe-Bildung) (Pseudomonas)

### Gewebeschaden:

Je größer die geschädigte Gewebemasse ist, desto langwieriger ist der Regenerationsprozeß. Das geschädigte Gewebe bietet - je massiver die Schädigung ist und je länger sie besteht - ein Nährmedium für Mikroorganismen, die eine sekundäre Infektion und wiederum den Heilungsverlauf kompliziert oder gar verhindert. Bei der qualitativen Differenzierung der Gewebeschädigung bestehen stufenlose Übergänge von Permeabilitätsstörung der Kapillargefäße bis zum vollständigen Gewebeuntergang (Nekrose).

Die Behandlung oder die Prophylaxe der chronischen Wundheilungsstörung muß sich an dieser Trias orientieren und die Tatsache berücksichtigen, daß diese drei Faktoren sich gegenseitig begünstigen.

### Prophylaxe der Wundheilungsstörung:

Die Prophylaxe der Wundheilungsstörung ist eine noch nicht zufriedenstellend gelöste Aufgabe, zudem muß die prophylaktische Therapie der Prognose einer Wunde bereits unmittelbar nach ihrer Entstehung angepaßt sein. Dabei spielen verschiedenste Faktoren in Anlehnung an obengenannte Trias eine Rolle.

Bei unkomplizierten sauberen Schnittwunden besteht ein geringes Risiko der Wundheilungsstörung. Höheres Risiko korreliert hauptsächlich mit ausgeprägten Gewebeschädigungen (z.B. traumatische Quetschwunden), Einschleppung pathogener Keime (z.B. Bißwunden) und endogene Faktoren (z.B. Makro-/Mikroangiopathie).

Auch die Heilung von Operationswunden ist prognostisch von verschiedenen Faktoren abhängig. Diese sind - abgesehen von der richtigen Ausführung der standardisierten präoperativen Reinigung und Desinfektion - unter anderem die Größe der Wunde, Dauer der Operation, technische Schwierigkeiten und Ausmaß des Operationstrauma. Die erneute Eröffnung einer Operationswunde bei Revisionen vor abgeschlossener Primärheilung birgt ein besonders hohes Risiko.

Eine maximale prophylaktische Therapie ist in der klinischen Praxis nicht sinnvoll, da die einsetzbaren Mittel verschiedene Nebenwirkungen aufweisen. Antiseptika haben beispielsweise eine zytotoxische Potenz. Antibiotika können abgesehen von den substanzspezifischen Nebenwirkungen zur Selektion resistenter Bakterienstämme führen. Des weiteren sind häufige Verbandswechsel und spezielle Verbandstechniken mit hohem personellem und materiellem Aufwand verbunden.

Aus diesen Ausführungen wird deutlich, daß sich die Prophylaxe der Wundheilungsstörung schwierig gestaltet und nicht immer erfolgreich ist. Es wird weiterhin deutlich, daß Wundheilungsstörungen ein problematisches Krankheitsbild darstellen für welches noch keine optimale und/oder allgemein gültige Therapie/Prophylaxe existiert und somit Behandlungs-/Prophylaxemöglichkeiten, welche eine höhere/schnellere Heilungsquote bei gleichzeitig vertretbaren Kostenaufwand ermöglichen dringend benötigt werden.

In den letzten Jahrzehnten aber auch schon in früheren Jahrhunderten wurden unzählige Substanzgruppen auf ihre Effektivität in der Behandlung der Wundheilungsstörung untersucht und bei den meisten von ihnen in irgend einer Form eine günstige Wirkung postuliert. Das Spektrum reicht von einfachen natürlich vorkommenden organischen und anorganischen Verbindungen wie Zucker und Zinkoxid bis zu den kompliziertesten Kombinationspräparaten und den gänzlich synthetisch-chemisch hergestellten Produkten.

Die vorliegende Erfindung stellt ein Mittel zur topischen Verwendung zur Therapie oder Prophylaxe von Wundheilungsstörungen, unterstützt durch ihre Gewebsgängigkeit und Zerstörung der Keime bei gleichzeitiger Schonung des Gewebes in Form von hochkonzentrierten (d.h. von mindestens 90%) bzw. reinen Gemischen von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae zur Therapie oder Prophylaxe der Wundheilungsstörung bereit.

Ätherische Öle sind Gemische von bis zu 50 einzelner flüssiger, ölartiger, in Wasser schwer löslicher, leicht flüchtiger Kohlenstoffverbindungen mit sehr unterschiedlicher chemischer Natur (Aldehyde, Alkohole, Ester, Säuren, Terpene, Cumarine, Phenole, Lactone, Ketone, Phenylether Oxide). Sie werden von einer Vielzahl - ca. 30 % - der höheren Pflanzenfamilien produziert.
Die wichtigsten Komponenten der ätherischen Öle sind Terpene, im besonderen Monoterpene und Sesquiterpene, welche mengenmäßig bis zu 95% eines ätherischen Öles ausmachen und für seine Hauptwirkungen verantwortlich sind.
Von Bedeutung sind außerdem Acetogenine oder Polyketide vertreten als Alkane und Alkene, sowie deren Folgeprodukte, Phenylpropanderivate (Abkömmlinge der aromatischen Aminosäuren Phenylalanin, Tyrosin und Dihydroxyphenylalanin).

Als im erfindungsgemäßen Gemisch zu verwendende ätherische Öle sind sowohl die natürlichen ätherischen Öle, als auch synthetische (naturidentische) Produkte - bestehend aus einem oder der Kombination mehrerer Hauptinhaltstoffe des entsprechenden natürlichen Öles - zu verstehen. Besonders bevorzugt ist hierbei die Verwendung des jeweiligen natürlichen ätherischen Öles.

Empirisch festgestellte Wirkungen haben den ätherischen Ölen im Laufe von Jahrtausenden weltweit in der Volksmedizin ein breites Einsatzspektrum als Therapeutika und Genußmittel, sowie als Hilfsmittel in Kosmetika, Lebensmittel und Pflegemittel verschafft.

Ätherische Öle gelten zudem als toxikologisch gut verträglich für den Menschen. Vergiftungen durch übermäßige Ingestionen sind äußerst selten, da dies im Allgemeinen durch die Geruchs- und Geschmacksintensität verhindert wird. Es existieren bereits wissenschaftlich fundierte Daten zur Toxikologie, Pharmakokinetik und Pharmakodynamik diverser ätherischer Öle (1,3).

Zur antimikrobiellen bzw. antiseptischen Wirksamkeit ätherischer Öle im Allgemeinen liegen zahlreiche Studien vor, hierbei wurden Wirkungen gegen Bakterien (6,7,8,11,12,14), Pilze (19,18) und Viren (19,20) gefunden. Generell kann gesagt werden, daß die Wirksamkeit der einzelnen Essenzen spezifisch und selektiv ist, wobei die Wirkung konzentrationsabhängig ist und eine vergleichbare antiseptische Potenz gegen Bakterien, Viren und Pilze gleichermaßen besteht.

Eine Durchblutungssteigerung konnte In einer doppelblind randomisierten Studie von Hong C.Z. et al. (6) für ein Eukalyptus-Mentholpräparat nachgewiesen werden. Dabei zeigte sich eine signifikante Hyperämie nach lokaler Verabreichung des Testpräparates gegenüber einem Placebo. Die lokale Steigerung der Durchblutung wurde ebenfalls an Schleimhäuten bei oraler Aufnahme im Gastrointestinaltrakt beobachtet. Der Wirkungsmechanismus ist noch nicht geklärt.

Zudem wirken ätherische Öle bei lokaler Verabreichung stark analgetisch, analog zu den Lokalanästhetika hemmen sie die Bildung von Aktionspotentialen der Schmerzrezeptoren. Sämtliche von Siemieniuk A. et al. untersuchten Substanzen zeigten eine stärkere Wirkung als Lidocain (16). Andere Untersuchungen von Gobel H. et al. (2) zeigten, dass ätherische Öle lokal verabreicht auch über komplexe Stimulations-/ Hemmimpulse zentral analgetisch wirken.

Ätherische Öle besitzen dank ihrer Lipophilität eine ausgeprägte Permeabilität und können die Hautbarriere bzw. die oberflächliche infizierte oder nekrotische Schicht einer Wunde in höheren Konzentration überwinden. Damit können sie auch als Trägersubstanzen (Penetrationsförderer) für Arzneistoffe die Permeabilität steigern (13).

In der Schweizer Patentschrift CH 688 787 A5 werden kursorisch Zusammensetzungen mit antiseptischer, antimikrobieller, antiviraler, antibiotischer, bakterizider, bakteriostatischer, desinfizierender, fungizider, fungistatischer, antiparasitärer, entzündungshemmender, hautpflegender, juckreizmildernder, heilungs-, wachstums-, und/oder durchbtutungsfördernder Wirkung beschrieben. Jedoch handelt es sich hierbei im Gegensatz zu den erfindungsgemäßen Zusammensetzungen nicht um hochprozentige Zusammensetzungen zur Applikation auf offenen Wunden, sondern um niedrig konzentrierte Zusammensetzungen (1 bis 10%) etwa zur Verwendung als Repellent für Haustiere, als ungezieferabtötendes Shampoo für Haustiere, als Pfoten- oder Fellpflegemittel, sowie als Pflanzenschutzmittel und Insektenrepellent.

In der Wundbehandlung wurden Ätherischöl-Drogen allerdings bisher ausschließlich in Form von Tinkturen oder Lösungen (Kamillen-, Salbei- oder Pfefferminzextrakte) in sehr niedrigen Konzentration eingesetzt. Es existieren jedoch weder Hinweise auf eine günstige Wirkung von hochprozentigen Auszügen der Ätherischöl-Drogen noch von unverdünnten ätherischen Ölen in der Prophylaxe / Behandlung von Wundheilungsstörungen. Aufgrund der bekannten hautreizenden Wirkung auf intakter Haut galt nach allgemeiner Lehrmeinung vielmehr die topische Applikation von unverdünnten bzw. hochprozentigen ätherischen Ölen auf verletzter Haut als kontrainduziert bzw. wurde nicht in Erwägung gezogen. Als hochprozentig im Zusammenhang mit der vorliegenden Erfindung werden Konzentrationen von mindestens 90% verstanden. Besonders bevorzugt sind hierbei unverdünnte ätherische Öle.

Die vorliegende Erfindung stellt somit ein völlig neues Mittel mit höherer und schnellerer Heilungsquote zur topischen Behandlung von Wundheilungsstörungen zur Verfügung. Denn es wurde unerwarteterweise gefunden, daß mittels der topischen Applikation von Gemischen von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae sich Wundheilungssstörungen erfolgreicher und / oder effektiver und / oder kostensparender behandeln lassen als durch die heutigen üblichen Standardtherapien.

Die erfindungsgemäße Verwendung der Gemische von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae ist besonders vorteilhaft, da sich ätherische Öle durch ihre antiseptische Wirksamkeit in höherer Konzentration bei nicht nennenswerter zytotoxischer bzw. nekrotisierender Wirkung gegenüber herkömmlich verwendeten Antiseptika auszeichnen. Zudem führt die Steigerung der lokalen Durchblutung im Wundgebiet zur Beschleunigung regeneratorischer Mechanismen durch Bildung von Granulationsgewebe. Auch die Funktionen des Immunsystems (Migration der Makrophagen, Übertritt von Antikörpern) kommen besser zum Tragen. Die Hyperämisierung hat außerdem eine besondere Bedeutung in der Behandlung von schlecht durchblutetem, geschädigtem Gewebe, welches sich in der klinischen Praxis äußerst problematisch und langwierig darstellt. Die Analgesie ist ein Nebeneffekt, der gerade in der akuten Phase bedeutsam ist und sympathischen dystrophischen Reaktionen entgegenwirkt. Zusätzlich können die lipophilen ätherischen Öle, bedingt durch ihre hohe Gewebsgängigkeit, höhere Konzentrationen im Gewebe erreichen und ihre Wirksamkeit in für sonstige Antiseptika oder Antibiotika schlecht erreichbare Schichten entfalten. Die Erhöhung der Gefäßpermeabilität steigert die Exsudation bzw. die Wundsekretion und führt somit zur verbesserten Wunddrainage sowie zur Abstoßung der Wundkeime und deren toxischen Produkte. Nicht zuletzt sprechen auch die geringen Kosten der Behandlung mit ätherischen Ölen und die einfache Handhabung für ihre praktische Anwendbarkeit.

Die in den erfindungsgemäßen Gemischen zu verwendenden ätherischen Öle werden durch die dem Fachmann bekannten Destillationsverfahren gewonnen, wobei in erster Linie die Wasser-Destillation, die Wasser-/Dampfdestillation und die Dampfdestillation zu nennen sind. Weitere verwendete Gewinnungsverfahren sind das Enfleurage-Verfahren (Ölextraktion), die Extraktion mit organischen Lösungsmitteln, sowie die Kaltpressung (Auspreßverfahren). Die so erhaltenen Öle, v.a. die wasserdampfdestillierten Öle können anschließend durch fraktionierte Destillation über Kolonnen (Rektifikation) weiter aufgereinigt werden.

Besonders bevorzugt sind Gemische aus den folgenden fünf der obengenannten ätherischen Öle Aetheroleum Menthae piperitae, Aetheroleum Cajeputi, Aetheroleum Eucalypti, Aetheroleum Juniperi und Aetheroleum Gaultheriae, sowie Mischungen in welchen Aetheroleum Menthae piperitae, Aetheroleum Cajeputi, Aetheroleum Eucalypti die Hauptwirkstoffe bzw. die einzigen Wirkstoffe sind.

Die nachfolgenden Beispiele dienen zur Veranschaulichung der erfindungsgemäßen Verwendung.

### Beispiele

### Beispiel 1

Es wird folgendes Mischpräparat ätherischer Öle auf das erkrankte Weichteilgewebe (Haut mit Anhangsgebilde und Unterhautgewebe) bei Vorliegen einer Wundheilungsstörung appliziert:

| | |
|---|---|
| Aetheroleum Menthae piperitae | 53% |
| Aetheroleum Cajeputi | 21% |
| Aetheroleum Eucalypti | 21% |
| Aetheroleum Juniperi | 3% |
| Aetheroleum Gaultheriae | 2% |

Die Häufigkeit der Anwendung sollte zwischen einmal alle zwei Tage und zweimal täglich (in besonderen Fällen häufiger) liegen.

### Beispiel 2

Mischpräparate wie in den vorstehenden Beispielen beschrieben werden mit Glyzerin, Vaseline oder einer anderen dem Fachmann bekannten lipophilen hautpflegenden oder hautschonenden Substanz verdünnt und wie unter Beispiel 1 beschrieben appliziert. Dabei soll der Anteil des(r) ätherischen Öle(s) jedoch wie oben erwähnt mindestens 90% betragen.

Nach Solubilisierung mit Lösungsvermittlern wie z.B. Tween® 80, können die erfindungsgemäß zu verwendenden ätherischen Öle auch in hydrophilen Träger formuliert werden.

Selbstverständlich können die ätherischen Öle auch in anderen als den hier aufgeführten dem Fachmann bekannten Vehikeln und Grundlagen formuliert werden.

### Beispiel 3

Es wird das Präparat wie in den Beispielen 1 und 2 in gleicher Applikationsform auf Wunden aufgetragen - besonders solche, die ein erhöhtes Risiko der Wundheilungsstörung aufweisen - um dieser vorzubeugen. Die Häufigkeit der Anwendung sollte sich an die Höhe des Heilungsstörungsrisikos orientieren und die Therapie sollte bei günstigem Heilungsverlauf ausgeschlichen werden.

Die erfindungsgemäße Applikation erfolgt durch direktes Auftragen eines Flüssigkeitsfilmes mit entsprechend sichtbarer "Befeuchtung".
Besonders bevorzugt ist hierbei die Applikation mittels eines Behältnisses aus Glas oder Aluminium mit einem handelsüblichen Dosiersprühkopf wie z.B. in Bauer K et al., Pharmazeutische Technologie (1997), Seite 257, beschrieben.

Eine weitere bevorzugte Ausführungsform der Erfindung stellen (Semi)okklusivverbände dar, welche mindestens 5% der erfindungsgemäßen Gemische enthalten zur Prophylaxe oder Therapie von Wundheilungsstörungen von v.a. grossflächigen Wunden um eine Austrocknung der Wundflächen zu verhindern und eine Reepithelisierung zu unterstützen.
Unter Semi(okklusiv)verbänden sind v.a. Wundverbände / Wundabdeckungen unter Verwendung von z.B. (Polyurethan)folien, Hydrokolloiden, Hydrofasern, Gelen (wie Polyacrylamid-Agar-Agar-Gel), Hydrogelen, Schaumstoffen (wie Polyvinylalkoholformalschaum, Polyurethanweichschaum oder Polymethacrylatschaum) und / oder Alginaten zu verstehen, wie z.B. von Eich D und Stadler R in VASA 1999; 28:3-9 beschrieben.
Durch den Kammereffekt sind hier auch schon geringere Konzentrationen, d.h. mindestens 5%, ausreichend, es können aber auch die im Zusammenhang mit den lipophilen und hydrophilen Grundlagen angesprochenen Konzentrationen verwendet werden.

Zum Nachweis der Wirksamkeit der erfindungsgemäßen Anwendung wurde eine kontrollierte randomisierte klinische Vergleichsstudie (Phase II/III) zur Wirksamkeit, Verträglichkeit und Akzeptanz eines Mischpräparats ätherischer Öle mit der Zusammensetzung: Aetheroleum Menthae Piperitae 53%, Aetheroleum Cajeputi 21%, Aetheroleum Eucalypti 21%, Aetheroleum Juniperi 3% und Aetheroleum Gaultheriae 2% in der lokalen Therapie der Wundheilungsstörung infizierter und sekundär heilender Wunden durchgeführt.

Überraschenderweise zeigte sich entgegen der allgemein vorherrschenden Meinung - basierend auf der typischen Reizempfindung auf der gesunden Haut-, daß die erfindungsgemäßen Gemische von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae den Schmerzreiz in einer Wunde weder provozieren, noch einen bestehenden Wundschmerz intensivieren. Im Gegensatz ließen sich die Schmerzen in Wunden verhindern oder lindern.

Die Wirksamkeit der Gemische von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae entfalten sich signifikant, wenn diese in Destillatsform (rein) bzw. hoch konzentrierten Präparaten (mind. 90%) in der topischen Behandlung der Wundheilungsstörung der chronischen Wunden eingesetzt werden und führen zur schnelleren Abheilung solcher Wunden im Vergleich mit anderen Therapeutika.

Die antiseptische Wirksamkeit der Gemische von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae in höheren Konzentration (mind. 90%) bei nicht nennenswerter zytotoxischer bzw. nekrotisierender Wirkung zeichnet sie gegenüber herkömmlichen Antiseptika aus. Die Steigerung der lokalen Durchblutung im Wundgebiet führt zur Beschleunigung regeneratorischer Mechanismen durch Bildung von Granulationsgewebe. Auch die Funktionen des Immunsystems (Immigration der Makrophagen, Übertritt von Antikörpern) kommen besser zum Tragen. Die Hyperämisierung hat außerdem eine besondere Bedeutung in der Behandlung von schlecht durchblutetem, geschädigtem Gewebe, welches sich in der klinischen Praxis äußerst problematisch darstellt. Die Analgesie ist ein Nebeneffekt, der gerade in der akuten Phase bedeutsam ist und sympathischen dystrophischen Reaktionen entgegenwirkt. Durch ihre hohe Permeabilität können die lipophilen ätherischen Öle höhere Konzentrationen im Gewebe erreichen und ihre Wirksamkeit in für sonstige Antiseptika oder Antibiotika schlecht erreichbaren Schichten entfalten. Die Erhöhung der Gefäßpermeabilität steigert die Exsudation bzw. die Wundsekretion und führt somit zur verbesserten Wunddrainage sowie zur Abstoßung der Wundkeime und deren toxischen Produkten. Es wurde auch beobachtet, daß die erfindungsgemäßen Gemische von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae in höheren Konzentration (mind. 90%) trotz Steigerung der Durchblutung gleichzeitig eine Hemmung entzündlicher Reaktionen bewirken, was zur Herstellung des physiologischen Gleichgewichts führt. Dies wurde in der untenstehenden klinischen Studie untersucht und bestätigt.

### Klinische Studie

Eine prospektive kontrollierte, randomisierte (1:1) monozentrische Vergleichsstudie (Phase II/III) mit 100 männlichen und weiblichen ambulanten und stationären Patienten aller ethnischer Gruppen mit Wundheilungsstörungen im Alter ≥ 18 Jahre wurde durchgeführt. Die Randomisierung erfolgte in 2 Blöcke ä 50 Patienten. Die Studiendauer war auf 6 Wochen +/- 1 Woche festgelegt. Die Hauptuntersuchungszeitpunkte waren bei Aufnahme in die Studie, bei Feststellung der völligen Abheilung und 3 Monate nach völliger Abheilung.

Hauptzielkriterien waren die Zeit zwischen Therapiebeginn und Abheilung, der Prozentsatz der klinischen Heilungserfolge, das Weiterbestehen des Heilungserfolges in der Nachbeobachtung sowie die Notwendigkeit der Anwendung weiterer chirurgischer Maßnahmen

Nebenzielkriterien der Studie waren: das Auftreten von Wundrandnekrosen, die Subjektive Beurteilung und Akzeptanz der Patienten und des Personals, zusätzliche systemische Antibiotikagabe, Dauer der stationären und der ambulanten Behandlung, sowie unerwünschte Ereignisse, Allergien und Unverträglichkeiten.

Die Wunden der Studienteilnehmer ließen sich wie folgt charakterisieren: infizierte Operationswunden am Stamm und an den Extremitäten, infizierte traumatische Wunden, Abszesse und Phlegmonen, Dekubitalulzera, feuchte Gangräne sowie Ulcera cruris venosa.

In beiden Patientengruppen (Studienmedikation (Gruppe A) bzw. Standardtherapie (Gruppe B)) wurden chirurgische debridierende Maßnahmen bei klarer Indikation sowie Wundreinigung bei jedem Verbandswechsel vorgenommen.
Bei Gruppe A wurde das ätherische Öl bei oberflächlichen bzw. ulzerösen Wunden auf die Wundfläche und auf die Innenseite des Wundverbandes appliziert.
Bei tiefen bzw. fistelbildenden Wunden wurde in den Wundgrund und in die Fisteln instilliert. Als Begleitmedikation/-therapie war nur Wundzucker zulässig.

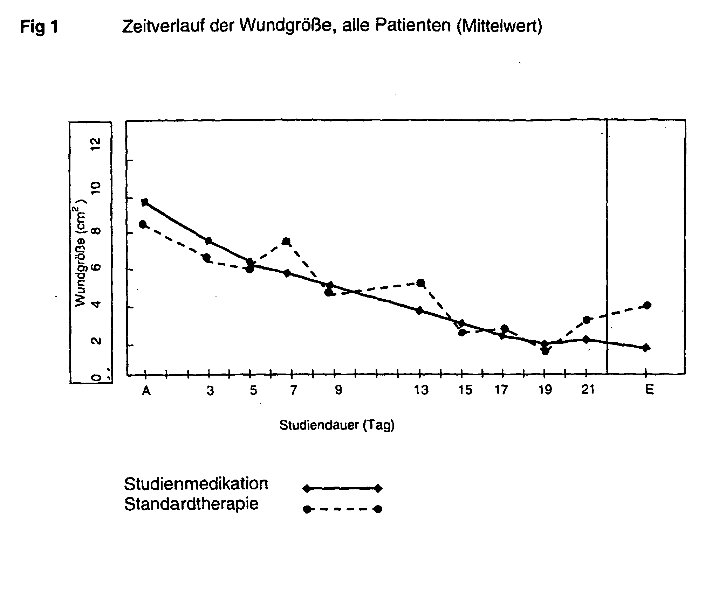

Gruppe B (Standardtherapie) wurde mit folgenden Präparaten behandelt: PVP-Jod lösung, PVP-Jod Salbe, Isopropanol, Fibrolan®, Viridase®, Iruxol®,Wundzucker oder Jodoform® Gaze.
In den folgenden Tabellen und Figur 1 bis sind die Ergebnisse der klinischen Studie zusammengefaßt.

**Tabelle 1**

| Veränderung im Bezug auf den Ausgangsbefund nach Ablauf der Studiendauer 6 Wochen ±1 Woche (Behandlungsergebnisse nach der vorgesehenen Studiendauer) | | | | | | |
|---|---|---|---|---|---|---|
| | Studienmedikation | | Standardtherapie | | Total | |
| | Anzahl der Patienten | in Prozent | Anzahl der Patienten | in Prozent | Anzahl der Patienten | in Prozent |
| offen | 13 | 24,5% | 23 | 46,0% | 36 | 35,0% |
| abgeheilt | 40 | 75,5% | 27 | 54,0% | 67 | 65,1% |
| Total | 53 | 100,0% | 50 | 100,0% | 103 | 100,0% |
| Fisher's exact test, p=0,019 | | | | | | |

**Tabelle 2:**

| Differenzierung nach Wundbefund bei der Entlassung | | | | | | |
|---|---|---|---|---|---|---|
| | Studienmedikation | | Standardtherapie | | Total | |
| | Anzahl der Patienten | in Prozent | Anzahl der Patienten | in Prozent | Anzahl der Patienten | in Prozent |
| Befund gleich oder schlechter | 5 | 9,4% | 10 | 20,0% | 15 | 14,6% |
| Befund kleiner | 8 | 15,1% | 13 | 26,0% | 21 | 20,4% |
| Abgeheilt | 40 | 75,5% | 27 | 54,0% | 67 | 65,0% |
| Total | 53 | 100,0% | 50 | 100,0% | 103 | 100,0% |

**Tabelle 3:**

| Stratifizierung der Patienten in Gruppen nach Wundart | | | | | | |
|---|---|---|---|---|---|---|
| | Studienmedikation | | Standardtherapie | | Total | |
| | Anzahl der Patienten | in Prozent | Anzahl der Patienten | in Prozent | Anzahl der Patienten | in Prozent |
| Sekundäre Wundheilung (Fisher's exact test p = 0,016) | | | | | | |
| Offen | 4 | 11,8% | 11 | 37,9% | 15 | 23,8% |
| abgeheilt | 30 | 88,2% | 18 | 62,1% | 48 | 76,2% |
| Total | 34 | 100,0% | 29 | 100,0% | 63 | 100,0% |

| Abszeß (Fisher's exact test p = 0,196) | | | | | | |
|---|---|---|---|---|---|---|
| Offen | 0 | 0,0% | 3 | 37,5% | 3 | 23,1% |
| abgeheilt | 5 | 100,0% | 5 | 62,5% | 10 | 76,9% |
| Total | 34 | 100,0% | 29 | 100,0% | 63 | 100,0% |

| Diabetisches Ulcus (Fisher's exact test p = 0,929) | | | | | | |
|---|---|---|---|---|---|---|
| Offen | 3 | 75,0% | 2 | 50,0% | 5 | 62,5% |
| abgeheilt | 1 | 25,0% | 2 | 50,0% | 3 | 37,5% |
| Total | 4 | 100,0% | 4 | 100,0% | 8 | 100,0% |

| Ulcus cruris venosum (Fisher's exact test p = 0,667) | | | | | | |
|---|---|---|---|---|---|---|
| Offen | 4 | 57,1% | 2 | 66,7% | 6 | 60,0% |
| abgeheilt | 3 | 42,9% | 1 | 33,3% | 4 | 40,0% |
| Total | 7 | 100,0% | 3 | 100,0% | 10 | 100,0% |

| AVK Stadium IIB - IV (Fisher's exact test p = 0,583) | | | | | | |
|---|---|---|---|---|---|---|
| Offen | 2 | 66,7% | 5 | 83,3% | 7 | 77,8% |
| abgeheilt | 1 | 33,3% | 1 | 16,7% | 2 | 22,2% |
| Total | 3 | 100,0% | 6 | 100,0% | 9 | 100,0% |

**Tabelle 4:**

| Patienten ohne Gefäßrisiko (keine erhebliche Störung der Durchblutung) | | | | | | |
|---|---|---|---|---|---|---|
| | Studienmedikation | | Standardtherapie | | Total | |
| | Anzahl der Patienten | in Prozent | Anzahl der Patienten | in Prozent | Anzahl der Patienten | in Prozent |
| offen | 4 | 10,3% | 14 | 37,8% | 18 | 23,4% |
| abgeheilt | 35 | 89,7% | 23 | 62,2% | 58 | 76,3% |
| Total | 39 | 100,0% | 37 | 100,0% | 76 | 100,0% |
| Fisher's exact test, p= 0,016 | | | | | | |

**Tabelle 5:**

| Patienten mit erheblichem Gefäßrisiko (fortgeschrittene Durchblutungsstörung) | | | | | | |
|---|---|---|---|---|---|---|
| | Studienmedikation | | Standardtherapie | | Total | |
| | Zahl der Patienten | In Prozent | Zahl der Patienten | In Prozent | Zahl der Patienten | In Prozent |
| Diab. Angiopathie | | | | | | |
| offen | 3 | 75,0% | 2 | 50,0% | 5 | 62,5% |
| abgeheilt | 1 | 25,0% | 2 | 50,0% | 3 | 37,5% |
| Total | 4 | 100,0% | 4 | 100,0% | 8 | 100,0% |

| Ulcus cruris venosum | | | | | | |
|---|---|---|---|---|---|---|
| offen | 4 | 57,1% | 2 | 66,7% | 6 | 60,0% |
| abgeheilt | 3 | 42,9% | 1 | 33,3% | 4 | 40,0% |
| Total | 7 | 100,0% | 3 | 100,0% | 10 | 100,0% |

| AVK Stad. IIB-IV | | | | | | |
|---|---|---|---|---|---|---|
| offen | 2 | 66,7% | 5 | 83,3% | 7 | 77,8% |
| abgeheilt | 1 | 33,3% | 1 | 16,7% | 2 | 22,2% |
| Total | 3 | 100,0% | 6 | 100,0% | 9 | 100,0% |
| | | | | | | |

| Alle | | | | | | |
|---|---|---|---|---|---|---|
| offen | 9 | 64,3% | 9 | 69,3% | 18 | 66,7% |
| abgeheilt | 5 | 35,7% | 4 | 30,7% | 9 | 33,3% |
| Total | 14 | 100,0% | 13 | 100,0% | 27 | 100,0% |

**Tabelle 6:**

| Patienten mit allgemeinen Risikofaktoren (systemische, maligne und endokrine Erkrankungen sowie Steroidbehandlung und Immunsuppressiva) | | | | | | |
|---|---|---|---|---|---|---|
| | Studienmedikation | | Standardtherapie | | Total | |
| | Anzahl der Patienten | in Prozent | Anzahl der Patienten | in Prozent | Anzahl der Patienten | in Prozent |
| offen | 4 | 22,2% | 10 | 58,8% | 14 | 40,0% |
| abgeheilt | 14 | 77,8% | 7 | 41,2% | 21 | 60,0% |
| Total | 18 | 34,0%* | 17 | 34,0%* | 35 | 34,0%* |

| | | | | | | |
|---|---|---|---|---|---|---|
| * % -Angabe ist auf die Gesamtzahl der Patienten bezogen | | | | | | |

**Tabelle 7:**

| Subjektive Beurteilung der Behandlung durch die Patienten | | | | | | |
|---|---|---|---|---|---|---|
| | Studienmedikation | | Standardtherapie | | Total | |
| | Anzahl der Patienten | in Prozent | Anzahl der Patienten | in Prozent | Anzahl der Patienten | in Prozent |
| Patientenzahl | 53 | 100,0% | 50 | 100,0% | 103 | 100,0% |
| keine Angaben | 0 | 0,0% | 1 | 2,0% | 1 | 1,0% |
| sehr gut | 20 | 37,7% | 0 | 0,0% | 20 | 19,2% |
| gut | 24 | 45,3% | 19 | 37,3% | 43 | 41,3% |
| mäßig | 6 | 11,3% | 23 | 47,1 % | 29 | 28,8% |
| schlecht | 3 | 5,7% | 7 | 13,7% | 10 | 9,6% |

Aus den obenstehenden Ausführungen geht deutlich hervor, daß die vorliegenden erfindungsgemäßen Gemische von aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae ein vorteilhaftes neues Arzneimittel zur topischen Behandlung von Wundheilungsstörungen, wie unter anderem infizierten Operationswunden am Stamm und den Extremitäten, infizierten traumatischen Wunden, Abszessen, Phlegmonen, Dekubitalulzera, feuchten Gangränen, Pyoderma gangraenosum, Ekthymata, superinfizierten Wunden, Säure- und Laugenverätzungen, Verbrennungen, sowie Ulcus cruris venosa, mit ätherischen Ölen zur Verfügung stellt.

In dieser Vergleichsstudie konnte bessere Wirksamkeit und Vorteile auf breiter Basis dieser erfinderischen Idee eines Indikationsgebiets medizinischer Behandlung gegenüber bisheriger Behandlungskonzepte gezeigt werden. Die Akzeptanz durch die Patienten ist hoch und somit die zu erwartende Compliance bei therapierefraktären Erkrankungen gut.

Zudem wurden bereits weitere positive Erfahrungen in Kombination mit modernen (Semi-)okklusivverbänden gemacht, was auf weitere Fortschritte in der Behandlung von Wundheilungsstörungen hoffen läßt.
Die niedrigen Behandlungskosten sind ein weiterer Vorteil dieses Behandlungskonzepts.
Weitere Erfahrungen zeigten auch besonders gute Wirksamkeit von hydrophilen und lipophilen Konzentraten (90%) der erfindungsgemäßen Gemische in der Behandlung bestimmter Formen der obengenannten Krankheitsbilder. Hierzu zählen vor allem solche Krankheitsbilder die entweder mit erheblichen Durchblutungsstörungen einhergehen, oder von ihnen verursacht werden.

Besonders bevorzugt und vorteilhaft ist hierbei die Applikation mittels Behältnisses mit Dosiersprühkopf. Hierdurch wird eine optimale lokale Dosierung erzielt und sonstiger Kontakt mit der Medikation fernab des erkrankten Organs vermieden.

### Literatur

1. Delgado IF, Carvalho RR, Nogueira AC, Mattos AP, Figueiredo LH, Oliveira SH, Chahoud I,
   Paumgartten FJ, Study on embryo-foetotoxicity of beta-myrcene in the rat. Food-Chem-Toxicol, Jan 1993 31 (1) 31-5
2. Göbel H, Schmidt G, Soyka D
   Effect of peppermint and eucalyptus oil preparations on neurophysiological and experimental algesimetric headache parameters.
   Cephalalgia, Jun 1994 14(3) 228-34
3. Guseinov D, Kagramanov KM, Kasumov F, Akhundov RA
   Research on the chemical composition and aspects of the pharmacological action of the essential oil of Kochi thyme (Thymus kotschyanus Boiss).
   Farmakol-Toxikol, Mar-Apr 1987 50(2) 73-74
4. Harkenthal M, Reichling J, Geiss HK, Saller R
   Comperative study on the in vitro antibacterial activity of Australien tee tree oil, cajuput oil, manuka oil, and eucalyptus oil. Pharmazie, 1999 54(6) 460-63
5. Wundheilung und Wundmanagement, Ein Leitfaden für die Praxis, R.A. Hatz, R. Niedner. W. Vanscheidt W. Westerhof, Springer-Verlag Berlin Heidelberg (1993) Seite 17ff)
6. Hong CZ, Shellock FG
   Effects of topically applied counterirritant (Eucalyptamint) on cutaneous blood flow and on skin and muscle temperatures. A placebo-controlled study. Am J Phys Med Rehabil, Feb 1991 70(1) 29-33
7. Jedlickova Z, Mottl O, Sery V
   Antibacterial properties of the vietnamese cajeput oil and ocimum oil in combination with antibactrial agents.
   J Hyg Epidemiol Microbiol Immunol (Czechoslovakia), 1992 36(3) 303-9
8. Kalodera Z, Pepeljnjak S, Vladimir S, Blazevic N
   Antimicrobial activity of essential oil from Micromeria thymifolia.
   Pharmazie, May 1994 49(5) 376-7
9. Kishore N, Mishra AK, Chansouria JP
   Fungitoxicity of essential oils against dermatophytes.
   Mycoses, May-Jun 1993 36(5-6) 211-5
10. Langezaal CR, Chandra A, Scheffer JJ
   Antimicrobial screening of essential oils and extracts on some Humulus lupulus L. cultivars.
   Pharm Weekbl Sci, Dec 11 1992 14 (6) 353-6
11. Marsh PD
   Microbiological aspects of the chemical control of plaque and gingivitis J Dent Res, Jul 1992 71(7) 1431-8
12. Moleyar V, Narasimham P
   Antibacterial activity of essential oil components
   Int J Food Microbiol, Aug 1992 16(4) 337-42
13. Obata Y, Takayama K, Okabe H, Nagai T
   Effect of cyclic monotetpenes on percutaneous absorption in the case of a water-soluble drug (diclofenac sodium).
   Drug Des Deliv, Oct 1990 6(4) 319-28
14. Panizzi L, Flamini G, Cioni PL, Morelli I
   Composition and antimicrobial properties of essential oils of four Mediterranean Lamiaceae.
   J Ethnopharmacol, Aug 1993 39(3) 167-70
15. Saracoglu I, Inoue M, Calis I, Ogihara Y
   Studies on constituents with cytotoxic and cytostatic activity of two Turkish medicinal plants Phlomis armeniaca and Scutellaria salviifolia.
   Biol Pharm Bull, Oct 1995 18(10) 1396-400
16. Wundheilung von Karel M. Sedlarik, 2. Auflage Gustav Fischer Verlag (1993) Seite 165 ff
17. Siemieniuk A, Szalkowska-Pagowska H, Lochynski S, Piatkowski K, Filipek B, Krupinska J, Czarnecki R, Librowski T, Zebala K
   Synthesis and local anesthetic and circulatory actions of aminoesters and hydroxyamine monoterpene derivatives.
   Pol J Pharmacol Pharm, Jul-Aug 1992 44(4) 407-20
18. Tong MM, Altman PM, Bametson R S
   Tea tree oil in the treatment on tinea pedis
   Australas-J-Dermatol, 1992 33 (3) 145-9
19. Armaka M, Papanikolaou E, Sivropoulou A, Arsenakis M
   Antiviral properties of isoborneol, a potent inhibitor of herpes virus simplex type 1.
   Antiviral Res, 1999 Sep 43(2) 79-92
20. Hayashi K, Kamiya M, Hayashi T
   Virucidal effects of the steam distillate from Houttuynia cordata and its components on HSV-1, influenza virus and HIV.
   Planta Med, 1995 Jun 61 (3) 237-41

## Patentansprüche

1. Verwendung eines Gemisches aus mindestens 3 der nachstehenden ätherischen Öle: Aetheroleum Menthae piperitae, Aetheroleum Cajeputi, Aetheroleum Eucalypti, Aetheroleum Melaleucae, Aetheroleum Juniperi und Aetheroleum Gaultheriae in einer Konzentration von mindestens 90% zur Herstellung eines Arzneimittels zur Therapie oder Prophylaxe der Wundheilungsstörung.

2. Verwendung nach Anspruch 1, zur Prophylaxe oder Therapie bei Wundheilungsstörungen bei infizierten Operationswunden am Stamm oder den Extremitäten, infizierten traumatischen Wunden, Abzessen, Phlegmonen, Dekubitalulzera, Pyoderma gangraenosum, Ekthymata, superinfizierten Wunden, Säureverätzungen, Laugenverätzungen, Verbrennungen, feuchten Gangränen oder Ulcera cruris.

3. Verwendung nach einem der Ansprüche 1 oder 2, wobei das Gemisch unverdünnt angewendet wird.

4. Verwendung nach einem der Ansprüche 1 bis 3, wobei das Gemisch in Form wässriger oder öliger Lösungen vorliegt.

5. Verwendung nach Anspruch 4, wobei das Gemisch in Glycerin, Vaseline oder sonstiger hautpflegender Substanz verdünnt wird.

6. Verwendung nach Anspruch 4, wobei das Gemisch mit Hilfe eines geeigneten Lösungvermittlers wasserlöslich gemacht wird und in hydrophiler Lösung verdünnt wird.

7. Verwendung nach einem der Ansprüche 1 bis 6, wobei das Gemisch Aetheroleum Menthae piperitae, Aetheroleum Cajeputi und Aetheroleum Eucalypti enthält.

8. Verwendung nach einem der Ansprüche 1 bis 6, wobei das Gemisch Aetheroleum Menthae piperitae, Aetheroleum Cajeputi, Aetheroleum Eucalypti, Aetheroleum Juniperi und Aetheroleum Gaultheriae enthält.

9. Verwendung nach einem der Ansprüche 1 bis 6 und 8, wobei das Gemisch die folgenden Komponenten umfaßt:
| | |
|---|---|
| Aetheroleum Menthae piperitae | 53% |
| Aetheroleum Cajeputi | 21% |
| Aetheroleum Eucalypti | 21%. |

10. Verwendung nach einem der Ansprüche 1 bis 9, wobei das Gemisch die folgende Zusammensetzung aufweist:
| | |
|---|---|
| Aetheroleum Menthae piperitae | 53% |
| Aetheroleum Cajeputi | 21% |
| Aetheroleum Eucalypti | 21% |
| Aetheroleum Juniperi | 3% |
| Aetheroleum Gaultheriae | 2%. |

11. Verwendung nach einem der Ansprüche 1-10, wobei die Applikation mit einem Dosiersprühkopf erfolgt.

12. Verwendung von Semiokklusiv- oder Okklusiwerbänden, umfassend X mindestens eines der vorstehend definierten Gemische zur Herstellung eines Arzneimittels zur Prophylaxe oder Therapie der Wundheilungsstörung.

## Claims

1. Use of a mixture of at least three of the following ethereal oils: Menthae piperitae aetheroleum, Caeputi aetheroleum, Eucalypti aethereoleum, Melaleucae aetheroleum, Juniperi aetheroleum and Gaultheriae aetheroleum in a concentration of at least 90% for the preparation of a pharmaceutical composition for the treatment or prevention of the wound healing disorder.

2. Use according to claim 1 for the prevention or treatment of wound healing disorders in the case of infected surgical wounds at the truncus or at the limbs, infected traumatic wounds, abscesses, phlegmons, decubital ulcers, Meleney's ulcer, ecthyma, superinfected wounds, acid injuries, base injuries, burns, moist gangrenes or crural ulcer.

3. The use of any one of claims 1 or 2 wherein the mixture is used undiluted.

4. The use of any one of claims 1 to 3 wherein the mixture is present in the form of aqueous or oily solutions.

5. The use according to claim 4 wherein the mixture is diluted in glycerine, paraffin jelly, or another skin-care substance.

6. The use according to claim 4 wherein the mixture is rendered water-soluble by means of a suitable solubiliser and is diluted in a hydrophilic solution.

7. The use according to any one of claims 1 to 6 wherein the mixture contains Menthae piperitae aetheroleum, Caeputi aetheroleum and Eucalypti aetheroleum.

8. The use of any one of claims 1 to 6 wherein the mixture contains Menthae piperitae aetheroleum, Caeputi aetheroleum, Eucalypti aethereoleum, Juniperi aetheroleum and Gaultheriae aetheroleum.

9. The use of any one of claims 1 to 6 and 8 wherein the mixture contains the following components:
| | |
|---|---|
| Menthae piperitae aetheroleum | 53% |
| Caeputi aetheroleum | 21% |
| Eucalypti aetheroleum | 21%. |

10. The use of any one of claims 1 to 9 wherein the mixture has the following composition:
| | |
|---|---|
| Menthae piperitae aetheroleum | 53% |
| Caeputi aetheroleum | 21% |
| Eucalypti aetheroleum | 21% |
| Juniperi aetheroleum | 3% |
| Gaultheriae aetheroleum | 2%. |

11. The use of any one of claims 1 to 10, wherein the application is carried out with a dosing spray head.

12. The use of semi occlusive or occlusive dressings, comprising at least one of the mixtures defined above for the preparation of a pharmaceutical composition for the prevention or treatment of the wound healing disorder.

## Revendications

1. Utilisation d'un mélange d'au moins 3 des huiles éthérées suivantes : Aetheroleum Menthae piperitae, Aetheroleum Cajeputi, Aetheroleum Eucalypti, Aetheroleum Melaleucae, Aetheroleum Juniperi et Aetheroleum Gaultheriae en une concentration d'au moins 90 % pour la préparation d'un médicament pour la thérapie ou la prophylaxie de troubles de la cicatrisation.

2. Utilisation selon la revendication 1, pour la prophylaxie ou la thérapie de troubles de la cicatrisation lors de plaies opératoires infectées du tronc ou des extrémités, de plaies traumatiques infectées, d'abcès, de flegmons, d'ulcères de décubitus, de Pyoderma gangraenosum, d'Ekthymata, de plaies surinfectées, de brûlures dues à des acides, de brûlures dues à des bases, de brûlures dues au feu, de gangrènes humides ou d'Ulcera cruris.

3. Utilisation selon l'une quelconque des revendications 1 ou 2, dans laquelle le mélange est utilisé non dilué.

4. Utilisation selon l'une quelconque des revendications 1 à 3, dans laquelle le mélange se présente sous forme d'une solution aqueuse ou huileuse.

5. Utilisation selon la revendication 4, dans laquelle le mélange est dilué dans de la glycérine, de la vaseline ou autres substances destinées au soin de la peau.

6. Utilisation selon la revendication 4, dans laquelle le mélange est rendu soluble dans l'eau à l'aide d'un agent de solubilisation approprié et dilué dans une solution hydrophile.

7. Utilisation selon l'une quelconque des revendications 1 à 6, dans laquelle le mélange contient Aetheroleum Menthae piperitae, Aetheroleum Cajeputi et Aetheroleum Eucalypti.

8. Utilisation selon l'une quelconque des revendications 1 à 6, dans laquelle le mélange contient Aetheroleum Menthae piperitae, Aetheroleum Cajeputi, Aetheroleum Eucalypti, Aetheroleum Juniperi et Aetheroleum Gaultheriae.

9. Utilisation selon l'une quelconque des revendications 1 à 6 et 8, dans laquelle le mélange comprend la composition suivante :
| | |
|---|---|
| Aetheroleum Menthae piperitae | 53 % |
| Aetheroleum Cajeputi | 21 % |
| Aetheroleum Eucalypti | 21 % |

10. Utilisation selon l'une quelconque des revendications 1 à 9, dans laquelle le mélange présente la composition suivante :
| | |
|---|---|
| Aetheroleum Menthae piperitae | 53 % |
| Aetheroleum Cajeputi | 21 % |
| Aetheroleum Eucalypti | 21 % |
| Aetheroleum Juniperi | 3 % |
| Aetheroleum Gaultheriae | 2 %. |

11. Utilisation selon l'une quelconque des revendications 1 à 10, dans laquelle l'application a lieu au moyen d'une tête de vaporisation pour dosage.

12. Utilisation de bandages semi-occlusifs ou occlusifs comprenant au moins un des mélanges précédemment définis pour la fabrication d'un médicament pour la prophylaxie ou la thérapie de troubles de la cicatrisation.
